# EUROPEAN PATENT APPLICATION

(11) **EP 3 974 811 A1**
(43) Date of publication of application: **30.03.2022**
(21) Application number: 20198942.3
(22) Date of filing: 29.09.2020
(51) Int. Cl.: G01N 21/31, B01L 3/00, G01N 21/64, G01N 33/487, G01N 21/03

(54) **DETERMINATION OF PARAMETERS IN BIOLOGICAL FLUIDS USING FLUORESCENCE AND ABSORPTION**

(71) Applicant: ProBiomics GmbH, 12205 Berlin (DE)
(72) Inventor: SCHWEIGERT, Florian, 12205 Berlin (DE)
(74) Representative: Patentanwälte Ruff, Wilhelm, Beier, Dauster & Partner mbB

(57) **Abstract**

A device for the optical analysis of parameters in biological fluids, in particular in blood, includes first means for photometric determination of at least one first parameter, in particular of hemoglobin (Hb), and second means for determination of at least one second parameter, in particular of zinc protoporphyrin (ZPP) by fluorescence measurement.

Both the first means and the second means include a light source and a light detector.

Preferably the second means are designed for auto-fluorescence measurement and/or front face-fluorescence measurement.

## Description

This invention is related to a device and a method for the optical analysis of parameters in biological fluids, in particular in blood.

There is a great need to determine parameters of biological fluids, in particular to determine the components of said biological components qualitatively and quantitatively.

Examples of such biological fluids are saliva, urine, milk and especially blood. Relating to the biological fluid blood, the parameters of whole blood (untreated or treated), of blood serum or blood plasma are to be determined. All biological fluids, including blood, can come from animals and humans.

In this context it is known to determine the content of hemoglobin (Hb) in blood by a photometric measurement. A photometer is an instrument that measures the strength of electromagnetic radiation in the range from ultraviolet to infrared, including the visible spectrum. The person skilled in the art is familiar with those instruments. Devices for rapid testing of hemoglobin or for point-of-care testing of hemoglobin are available.

Another known option for determining parameters in biological fluids, in particular in blood, is fluorescence measurement. Fluorescence spectroscopy analyzes fluorescence from a sample. Usually ultraviolet light is used for exciting electrons in components of the biological sample, resulting in the emittance of light, in particular in the UV and visible light region. With this sensitive technique components containing one or more fluorescent chemical groups can be detected. Intrinsic protein fluorescence is due to the aromatic amino acids, mainly tryptophan, but can also be observed in vitamins, mycotoxins, bilirubin and advanced glycated proteins. A fluorescent component of blood is zinc protoporphyrin (ZPP).

Instruments for detecting zinc protoporphyrin (ZPP) by fluorescence measurement are available. However, according to the knowledge of the inventors, a non-laboratory based testing/point-of-care device for zinc protoporphyrin is not available.

Under the circumstances, the invention has the object to provide a device for the optical analysis of parameters in biological fluids, which allows the determination, in particular the quantitative determination, of at least two parameters/components in this biological fluid. Preferably, it should be possible to determine these at least two parameters together, i.e. in one measuring process or measuring cycle.

In this context, the new device preferably should be suitable for the determination of at least two parameters in blood, in particular in whole blood. Here, it is important to quantitatively determine the hemoglobin content and the zinc protoporphyrin content in blood with the new device. Further, the measured Hb data and the measured ZPP data are to be used to calculate the ZPP value in relationship to the Hb value.

The above-mentioned objects and other objects are solved by the device with the features of claim 1 and by the method with the features of claim 14. Preferred embodiments of the inventive device and the inventive method are defined in the corresponding dependent claims.

The wording of all claims is hereby incorporated into the description by explicit reference.

According to the invention the new device comprises first means for photometric determination of at least one first parameter, including at least one first light source and at least one first light detector. Preferably, said first parameter is hemoglobin (Hb).

Further, the new device comprises second means for determination of at least one second parameter by fluorescence measurement, including at least one second light source and at least one second light detector. Preferably, said second parameter is zinc protoporphyrin (ZPP).

The terms mentioned in context with the definition of the inventive device are defined as follows.

"Biological fluid" is a generic term for any bio-organic fluid produced by an organism. In the context of this invention biological fluids produced by animals and humans are of specific importance. Examples for such biological fluids are blood, blood serum, blood plasma, urine, saliva, interstitial fluid, cytosol, and others like milk.

"Photometric determination" (spectrophotometric analysis) is the determination (qualitatively or quantitatively) of substances by measuring their capacity to absorb light of various wavelengths.

"Fluorescence measurement" (fluorescence spectroscopy) is the (qualitative or quantitative) determination of substances including fluorophore molecules by exciting those molecules and by measuring the emitted fluorescence.

"Hemoglobin (Hb)" is a metalloprotein in the red blood cells (erythrocytes) which contains iron and is responsible for the oxygen transport in almost all vertebrates.

"Zinc protoporphyrin (ZPP)" is a compound found in red blood cells, if the generation of hemoglobin is inhibited by lead and/or by lack of iron. ZPP is an established indicator for iron deficiency, estimated to cause more than 50% of anemia cases.

It is preferred according to the invention that the first light source and/or the second light source comprise at least one light-emitting diode (LED).

As it is well known, a LED is a semiconductor light source that emits light if current flows through it. The energy which is required for electrons to cross the band gap of the semiconductor determines the energy of the emitted photons, and consequently the color of the emitted light. LEDs are generally classified into three wavelength ranges, namely ultraviolet, visible and infrared. Specific LEDs are available for specific applications, covering the necessary wavelength ranges.

Referring to the first light source, it is preferred that it emits light with a wavelength between 400 nm and 850 nm, preferably between 400 nm and 700 nm (for the photometric determination), in particular light with a wavelength between 500 nm and 600 nm.

Referring to the second light source, it is preferred that it emits light with a wavelength between 295 nm and 450 nm for fluorescence measurement, in particular light with a wavelength between 390 nm and 430 nm, preferably between 405 nm and 425 nm.

Further, it is preferred according to the invention that the first light detector and/or the second light detector comprise at least one photodiode or at least one photomultiplier. Said photomultiplier can preferably be at least one silicon photomultiplier (SiPM).

Photodetectors are devices used for the detection of light. They usually deliver an electronic output signal, e.g. a voltage or electric current. Photodiodes are semiconductor devices absorbing light and generating a photocurrent. Photomultipliers are specific phototubes (vacuum tubes or gas-filled tubes exploiting the photoelectric effect) with very high sensitivity. In this context SiPMs show, among others, low excess noise and are easy to use. Silicon photomultipliers (SiPMs) are very well suited for portable applications.

Referring to the first light detector and/or the second light detector, it is preferred if they are capable of detecting light with a wavelength between 250 nm and 1400 nm, preferably between 280 nm and 800 nm. For the detection of specific wavelength or wavelength ranges suitable filters are provided.

It is further preferred according to the invention if the second means (for fluorescence measurement) are designed for auto-fluorescence measurement.

It is possible to determine second parameters in a biological fluid by adding a further component which interacts with a component of the biological fluid, and generates fluorescence under excitation (secondary fluorescence).

However, it is preferred under the invention that the inventive device determines second parameters by auto-fluorescence measurement, originating from fluorophores which are already part of the biological fluid, like tryptophan, bilirubin, vitamins, glycated proteins and preferably zinc protoporphyrin (ZPP).

According to the invention, a device is also further preferred, in which the second means are designed for front face-fluorescence measurement.

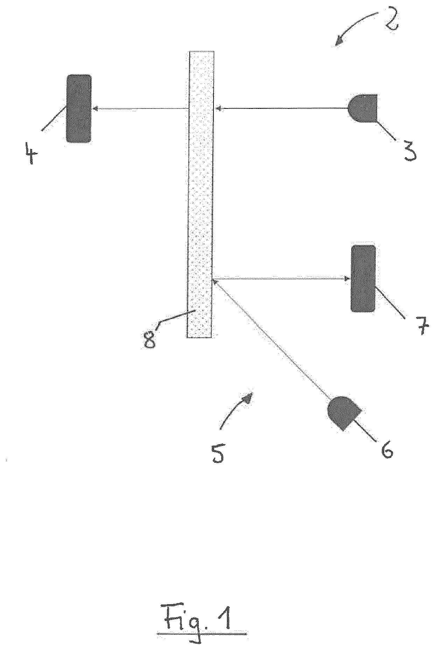

While it is possible to measure fluorescence in a right-angle geometry, wherein the exciting light and the emitted light enclose an angle of 90°, it is preferred to carry out the measurement in a geometry under an angle clearly below 90°, wherein the exciting light and the emitted light enter and leave the same sample face, i.e. the "front face" of the sample. The corresponding angle, which can be taken from Figure 1, preferable is in the range between 20 and 70°. As a consequence, the excitation light penetrates only a short distance of the sample.

According to the invention measures can be taken that the photometric measurement and the fluorescence measurement do not interfere with each other. This topic can be particularly important in relation to the two light detectors of the inventive device. In particular, if emission signals of components with auto-fluorescence are detected having (very) low signal intensity, it is preferred to place the first light detector and the second light detector at a distance from each other, or preferably to provide shielding means/shielding elements between the first light detector and the second light detector.

In another preferred embodiment of the inventive device, the device further includes an evaluation unit, which is capable of processing the data resulting from the photometric measurement and/or from the fluorescence measurement provided by the inventive device. This evaluation unit can process data which are directly related to the parameters which are (directly) measured in the device. Preferably, this evaluation unit can also compute further parameters from the (directly) determined parameters, in order to provide even further parameters to the user of the device.

In particular, those further parameters can be evaluated/computed from blood parameters which are determined in the device, preferably from the parameters hemoglobin (Hb) and zinc protoporphyrin (ZPP). E.g. it is known to a person skilled in the art that the parameter/value of hematocrit (Ht) can be computed from the (determined) parameter/value of hemoglobin (Hb) according to a known mathematical formula.

In another preferred embodiment of the inventive device this device further includes a display. This display is in particular for displaying the determined and evaluated parameters mentioned earlier. E.g. the blood parameters hemoglobin (Hb) and/or zinc protoporphyrin (ZPP) and/or a ratio of these two parameters and a computed hematocrit (Ht) parameter can be shown to the user of the device in said display. To view all these parameters for the user of the device is especially important, if the inventive device is a point-of-care instrument.

Usually the sample for optical analysis, comprising the biological fluid, in particular the blood, will be presented to the inventive device or will be inserted into the inventive device on or in any kind of sample (object) carrier, sample slide or sample receptacle.

Nevertheless, according to the invention it is preferred that the sample is presented to or inserted into the device in a cuvette, in particular a microcuvette. While a cuvette usually has greater dimensions and holds greater volumes of the sample, a microcuvette only holds smaller volumes of the sample, e.g. volumes between 5 and 100 µL, preferably between 5 and 30 µL. Usually the (fluid) sample, namely the corresponding biological fluid, is introduced into the microcuvette via capillary forces.

As a consequence, the inventive device preferably includes a holder for a cuvette, in particular a microcuvette, wherein the holder is capable of introducing the cuvette and the biological fluid into the device. Preferably the holder is reversibly insertable into the device and/or is movable in relation to the device. With these embodiments it is possible to move the cuvette and the measuring section of the cuvette in relation to the first means and/or the second means which are parts of the inventive device.

The movability of the holder and the holder with the inserted cuvette/microcuvette is also of importance because it allows making the corresponding measurements at multiple sites of the cuvette/microcuvette over time.

The movement of the holder with the inserted cuvette/microcuvette along at least one axis relating to the first and second measuring means can be done in a way that the cuvette/microcuvette with its measuring window(s) or its measuring section(s) passes the two means with their light sources and detectors, and during this passage multiple measurements can be made and the corresponding data can be taken from the same sample. Thus, average values of the measurement data can be calculated. This allows for an improved accuracy of the data, and it also allows for the evaluation of the fact whether the cuvette/microcuvette is properly filled with the corresponding biological fluid, in particular blood. This is especially important, because any inhomogeneity or any air bubbles in the cuvette/microcuvette can greatly affect the values of the measurement.

The holder for the cuvette/microcuvette can be moved by a number of means which are known from the prior art. E.g. the holder can be moved mechanically, magnetically or by electronic forces. It is possible to push and retract the holder by hand, by using a spring mechanism, by using magnets or by using motors.

In the context of these explanations, it is possible to e.g. vary the number of photometric measurements and the number of fluorescence measurements. Preferably, the filling state of a cuvette/microcuvette can be analyzed by a number of photometric measurements at several sites on the measuring section of the cuvette/microcuvette. If the data from these photometric measurements are not acceptable, other sites of the measuring section can be evaluated, or the corresponding cuvette/microcuvette can be discarded.

If the data from the photometric measurements are fine, the corresponding fluorescence measurements can be taken from one or more sites of the measuring section of the cuvette/microcuvette.

Still, with the inventive device a first parameter and a second parameter are determined together in one sample in one measuring process or in one measuring cycle, by photometric measurement and fluorescence measurement, respectively.

As mentioned above, preferably a microcuvette is used for holding the sample (biological fluid) during measurement in the inventive device. Preferably, said microcuvette is a flat microcuvette, namely a microcuvette in which one of the three dimensions is considerably smaller than the other two dimensions.

The microcuvette has at least one measuring section, wherein the measuring section has two opposite, essentially planar surfaces which are arranged preferably substantially parallel to each other and have dimensions of at least 4 mm, preferably of at least 5 mm in both surface directions x and y.

It is preferred according to the invention that the device is portable, i.e. can be easily carried and handled by a user, even by a user with low strength. In this context, the inventive device can have at least one of the following preferred features, and preferably a combination of all these three features:
- a weight of less than 1 kg,
- dimensions of less than 20 cm,
- operated with rechargeable batteries.

In this context, it is further preferred, if the inventive device
- has dimensions of 17 cm x 17 cm x 5 cm,
- has a weight of approx. 0.5 kg,
- includes rechargeable Lithiumbatteries.

The invention further comprises a method for the optical analysis of parameters in biological fluids, in particular in blood. This method is characterized in that at least one first parameter and at least one second parameter are determined in one device, and preferably in one biological fluid sample, by photometric measurement and by fluorescence measurement, respectively. Preferably, said method is used with blood as the (one) biological fluid sample.

With respect to further preferred features of the inventive method reference is made to the features mentioned above in the context of the inventive device. In particular, it is preferred in the inventive method that the first parameter is the hemoglobin (Hb) content, and/or that the second parameter is the zinc protoporphyrin (ZPP) content.

One of the most preferred features of the inventive method is that the at least one first parameter and the at least one second parameter are determined together, preferably in one (biological fluid) sample.

The advantages of the inventive device and of the inventive method are evident from the above disclosure.

With the inventive device at least two parameters in a biological fluid sample, in particular in a blood sample, can be determined together in one measuring process or measuring cycle, in particular quantitatively and in particular simultaneously. A complex sample preparation is not necessary. Quite to the contrary, the biological fluid, in particular the blood, can be directly brought into the sample receptacle, in particular the cuvette/microcuvette.

The device is suitable for point-of-care application, in particular for the measurement of blood values, preferably of hemoglobin (Hb) and zinc protoporphyrin (ZPP). Therefore, the device is optimized for point-of-care screening of anemia and iron deficiency.

Rapid measurements within less than 10 minutes, even of less than 5 minutes, are possible, with results equivalent to laboratory bound tests.

Further advantages and features of the invention will become clear from the following description of a drawing and of an example in conjunction with the dependent claims. The individual features can be realized either singly or in combination in one embodiment of the invention. The drawing and the example merely serve for illustration and better understanding of the invention and are not to be understood as in any way limiting the invention.

The figure schematically shows:
- Fig. 1: the setup of an inventive device.

Fig. 1 shows the arrangement of the first means 2 and the second means 5 with respect to each other and with respect to a cuvette/microcuvette 8. First means 2 include a (first) light source 3 and a (first) light detector 4.

Second means 5 include a (second) light source 6 and a (second) light detector 7. Second means 5 are designed for front face-fluorescence measurement. Therefore, the exciting light from second light source 6 and the emitted light going to the second light detector 7 enter and leave the same sample face (of cuvette 8) in a geometry with an angle clearly below 90°.

It should be clear from Fig. 1 that the shown setup is not limiting to the invention, but should only explain the fact that according to the invention first means 2 for photometric measurement and second means 5 for fluorescence measurement are included in one device.

Further, Fig. 1 should not result in any limitation how the inventive device is used, in particular according to the inventive method. As explained in the description, the device can be used in various ways, also in accordance with the inventive method, e.g. by moving the cuvette in a holder, whereby various measuring sites are exposed to the first means 2 and the second means 3 successively or simultaneously.

### Example

A portable inventive device having dimensions of approximately 17 cm x 17 cm x 5 cm, having a weight of approximately 0.5 kg, and including rechargeable batteries, is provided.

The device comprises a holder in which a microcuvette can be inserted. The holder with the microcuvette is movable, preferably inside the device, relating to first measuring means for photometric measurement and relating to second measuring means for fluorescence measurement.

For the determination of hemoglobin (Hb) and zinc protoporphyrin (ZPP) blood samples are transferred into microcuvettes by capillary forces, and the blood values of hemoglobin and zinc protoporphyrin are determined in these blood samples. With the aid of an evaluation unit and via a display the blood values of hemoglobin and zinc protoporphyrin and the ratio of these two measured blood values are presented to the user of the device.

In the example blood samples of approximately 15 µL volume are used for determination of the corresponding hemoglobin and ZPP values.

By comparison of the corresponding result with data from available devices for photometric measurement and fluorescent measurement it could be shown that the measurements with the inventive device are accurate. As a consequence, the inventive device is capable of determining the hemoglobin value and the zinc protoporphyrin value in one blood sample in one measuring process/measuring cycle.

## Claims

1. Device for the optical analysis of parameters in biological fluids, in particular in blood, comprising
• first means (2) for photometric determination of at least one first parameter, in particular of hemoglobin (Hb), including at least one first light source (3) and at least one first light detector (4), and
• second means (5) for determination of at least one second parameter, in particular of zinc protoporphyrin (ZPP), by fluorescence measurement, including at least one second light source (6) and at least one second light detector (7).

2. Device according to claim 1, **characterized in that** the first light source and/or the second light source comprise at least one light-emitting diode (LED).

3. Device according to claim 1 or claim 2, **characterized in that** the first light source emits light with a wave length between 400 nm and 850 nm, preferably between 400 nm and 700 nm, in particular light with a wave length between 500 nm and 600 nm, and/or the second light source emits light with a wave length between 295 nm and 450 nm, in particular light with a wave length between 390 nm and 430 nm, preferably between 405 nm and 425 nm.

4. Device according to anyone of the preceding claims, **characterized in that** the first light detector and/or the second light detector comprise at least one photo diode or at least one photomultiplier, preferably at least one silicon photomultiplier (SiPM).

5. Device according to anyone of the preceding claims, **characterized in that** the first light detector and/or the second light detector are capable to detect light with a wavelength between 250 nm and 1400 nm, preferably light with a wavelength between 280 nm and 800 nm.

6. Device according to anyone of the preceding claims, **characterized in that** the second means (5) are designed for auto-fluorescence measurement.

7. Device according to anyone of the preceding claims, **characterized in that** the second means (5) are designed for front face-fluorescence measurement.

8. Device according to anyone of the preceding claims, **characterized in that** the device further includes an evaluation unit, in particular for evaluating at least one further parameter from the determined parameters, in particular from the blood parameters of hemoglobin (Hb) and/or zinc protoporphyrin (ZPP).

9. Device according to anyone of the preceding claims, **characterized in that** the device further includes a display, in particular for displaying the determined and evaluated parameters, in particular the blood parameters hemoglobin (Hb) and/or zinc protoporphyrin (ZPP) and/or the further parameters evaluated from the determined parameters, in particular from the blood parameters hemoglobin (Hb) and/or zinc protoporphyrin (ZPP).

10. Device according to anyone of the preceding claims, **characterized in that** the device further includes a holder for a cuvette, wherein preferably the holder is reversibly insertable into the device and/or is movable in relation to the device.

11. Device according to claim 10, **characterized in that** the holder is designed to reversibly receive a microcuvette with at least one measuring section, the measuring section having two opposite, essentially planar surfaces which are arranged preferably substantially parallel to each other and having dimensions of at least 4 mm, preferably of at least 5 mm in both surface directions x and y.

12. Device according to anyone of the preceding claims, **characterized in that** the device is portable, in particular with a weight of less than 1 kg and/or with dimensions less than 20 cm and/or operated with rechargeable batteries.

13. Method for the optical analysis of parameters in biological fluids, in particular in blood, **characterized in that** at least one first parameter and at least one second parameter are determined together in one device, and preferably in one biological fluid sample, in particular in blood, by photometric measurement and by fluorescence measurement, respectively.

14. Method according to claim 13, **characterized in that** the first parameter is the hemoglobin (Hb) content, and/or that the second parameter is the zinc protoporphyrin (ZPP) content.

15. Method according to claim 13 or claim 14, **characterized in that** the at least one first parameter and the at least one second parameter are determined simultaneously, preferably in one sample.
